# NEUE EUROPÄISCHE PATENTSCHRIFT

(11) **EP 1 796 761 B2**
(45) Veröffentlichungstag und Bekanntmachung des Hinweises auf die Entscheidung über den Einspruch: **06.06.2018**
(45) Hinweis auf die Patenterteilung: 05.11.2008
(21) Anmeldenummer: 05807985.6
(22) Anmeldetag: 27.09.2005
(51) Int. Cl.: A61M 5/178, A61J 1/00, A61K 9/12, B01F 5/04, B01F 13/00, A61M 39/18

(54) **Vorrichtung zur Erzeugung eines medizinischen Schaums**
Device for producing a medical foam
Dispositif pour produire une mousse médicinale

(30) Priorität: 05.10.2004 DE 102004048749; 09.03.2005 DE 102005011174
(43) Veröffentlichungstag der Anmeldung: 20.06.2007
(73) Patentinhaber: CHEMISCHE FABRIK KREUSSLER & CO. GMBH, D-65203 Wiesbaden (DE)
(72) Erfinder: WOLLMANN, Jan-Christoph, 55545 Bad Kreuznach (DE)
(74) Vertreter: dompatent von Kreisler Selting Werner - Partnerschaft von Patent- und Rechtsanwälten mbB
(86) Internationale Anmeldenummer: PCT/EP2005/054819
(87) Internationale Veröffentlichungsnummer: WO 2006/037735

(56) Entgegenhaltungen:
- WO-A-2004/047969
- FR-A- 2 708 204
- US-A- 3 570 486
- US-A- 4 565 301
- US-A- 5 176 673
- US-A- 5 380 306
- US-A- 5 569 191
- US-A- 6 162 165
- US-A1- 2004 156 915
- US-B1- 6 471 671
- WOLLMANN J-C. G.R.: 'The history of sclerosing foams' THE AMERICAN SOCIETY OF DERMATOLOGIC SURGERY, INC. Bd. 30, Mai 2004, Seiten 694 - 703

## Beschreibung

Die Erfindung betrifft eine Vorrichtung zur Erzeugung von insbesondere reproduzierbarem medizinischem Schaum bzw. Bläschensuspension aus einem gasförmigen und einem flüssigen Medium. Insbesondere betrifft die Erfindung eine Mischvorrichtung für die reproduzierbare Zubereitung und Verabreichung von Injektabilia - wie z.B. Verödungsmittel, Diagnostika, Therapeutika, Homöopathika und Eigenblut.

Unter der Verödungstherapie versteht man die planvolle Ausschaltung von intra-, subkutanen und/ oder transfascialen Varizen sowie die Verödung subfascialer Gefäße bei venösen Fehlbildungen durch das Einspritzen eines Verödungsmittels. Die verschiedenen Verödungsmittel führen zu einer Schädigung des Endothels der Gefäße. Im Anschluss kommt es zu einem sekundären Gefäßverschluss und längerfristig zur Umwandlung der Venen in einen bindegewebigen Strang, zur Sklerose. Ziel der Verödungsbehandlung ist die definitive Umwandlung in einen fibrösen Strang. Dieser kann nicht rekanalisieren und entspricht in seinem funktionellen Ergebnis dem operativen Vorgehen zur Entfernung einer Varize. Neben der Verödung mit flüssigen Sklerosierungsmitteln gewinnt zunehmend die Verödung mit geschäumten Sklerosierungsmittein an Bedeutung. Der Schaum verbleibt länger in der Vene. Hierbei werden oberflächenaktive Verödungsmittel, wie z.B. Polidocanol, meistens durch Hin- und Herpumpen zwischen zwei Spritzen, durch Aufschütteln, durch Rühren oder mit Hilfe von Siebsystemen in einen schaumartigen Zustand gebracht und dann injiziert.

Ferner sind zahlreiche als Echokontrastmittel geeignete Zubereitungen bekannt, die z.T. oberflächenaktive Stoffe enthalten, die die Bildung von Bläschen unterstützen und diese stabilisieren. Die Ultraschall reflektierenden Bläschen bzw. ein Schaum sind das eigentliche Kontrastmittel und werden erst unmittelbar vor Verabreichung erzeugt.

Aus US 2004/0156915 ist es bekannt, ein Gas und einen flüssigen Wirkstoff in zwei gesonderten Behältern bereitzustellen. Der Gasbehälter steht unter Überdruck, so dass durch Verbinden der beiden Behälter Gas in den Flüssigkeitsbehälter strömt. Hierdurch entsteht in einem der beiden Behälter ein Gas-Flüssigkeits-Gemisch, wobei hierbei noch kein Mikroschaum entstehen soll. Anschließend können die beiden Behälter wieder voneinander getrennt werden und der Behälter, in dem sich das Gas-Flüssigkeits-Gemisch befindet, mit einer Spritze verbunden werden. Beim Ausströmen des Gas-Flüssigkeits-Gemischs in die Spritze wird ein Mikroschaum erzeugt, der sodann appliziert werden kann.

Aus WO 2004/047969 ist ein Doppelkolbensystem bekannt. Ein Spritzenauslass ist mit einem Kolben eines Behälters verbunden, in dem sich Gas befindet. Der Behälter ist fest mit einem zweiten Behälter verbunden, in dem sich Flüssigkeit befindet. Ferner ist die Spritzenöffnung mit einer Nadel verbunden, die innerhalb des Gasbehälters angeordnet ist. Durch Eindrücken der Spritze und somit des mit der Spritze verbundenen Kolbens in den Gasbehälter erfolgt durch die Nadel ein Öffnen des Flüssigkeitsbehälters, so dass Flüssigkeit aus dem Flüssigkeitsbehälter durch die Nadel in die Spritze strömt. Da gleichzeitig das Gas in dem Gasbehälter komprimiert wird, strömt durch Öffnungen in dem Kolben Gas in die Spritze. Durch das Mischen von Gas und Flüssigkeit beim Eintreten in die Spritze erfolgt die Erzeugung eines Schaums.

Eine Mischeinrichtung zur Herstellung von medizinischem Schaum bzw. zur Herstellung von Bläschen ist aus EP 0 564 505 bekannt. Hierin ist ein Mischer mit einem wendelförmigen Mischelement beschrieben. Bei dem Mischer handelt es sich um ein Zusatzelement, das mit einer Spritze unlösbar verbunden werden kann. Beim Herausdrücken von flüssigem und/oder gasförmigem Medium aus einer zweiten Spritze gelangt das Medium durch den Mischer in die andere Spritze, welche das Gas enthält. Hierbei werden Gasphase und Flüssigphase entlang des wendelfömigen Mischelements durchmischt. Hierdurch kann ein therapeutischer und/oder diagnostischer Schaum erzeugt werden. Bei dem Mischer handelt es sich um ein Bauteil, das auf Grund der in dem Mischer angeordneten spiralförmigen Mischelemente nur mit aufwändigen Spritzgussformen als Spritzgussteil hergestellt werden kann.

Insbesondere bei der Erzeugung von Schäumen zur medizinischen Verwendung, insbesondere für die Sklerotherapie, ist es erforderlich, einen sterilen Schaum zu erzeugen. Sofern zu der Schaumerzeugung Luft verwendet wird, ist es möglich, Luft durch einen Sterilfilter in eine Spritze einzusaugen und sodann die erzeugte sterile Luft zur Schaumerzeugung zu verwenden. Dies hat jedoch den Nachteil, dass zusätzliche Arbeitsschritte und ein zusätzliches Bauteil in Form des Sterilfilters erforderlich ist. Hierdurch werden die Kosten erhöht. Ferner wird die Abfallmenge vergrößert.

Ferner ist es aus GB 2 369 996 bekannt, eine Spritze, die mit steriler Luft gefüllt ist, mit einem Dreiwegchahn zu erschließen. Eine zweite Spritze wird mit einem Wirkstoff gefüllt und ebenfalls mit dem Drei-Wege-Hahn verbunden, so dass die Spritzen in einem 90°-Winkel zueinander angeordnet sind. Anschließend wird der Drei-Wege-Hahn in eine Stellung gedreht, in der die beiden Spritzen miteinander verbunden sind, so dass durch Hin- und Herpumpen des Gases und des Wirkstoffs ein Schaum erzeugt werden kann. Das Verschließen einer mit steriler Luft gefüllten Spritze mit einem Drei-Wege-Hahn weist den Nachteil auf, dass beispielsweise beim Transport oder beim Handling ein ungewolltes Öffnen und damit ein Kontaminieren und/ oder eine Veränderung der Gasmenge vorkommen kann. Des weiteren ist die Handhabung dieses Systems aufwändig, da nach dem Verbinden der beiden Spritzen mit dem Drei-Wege-Hahn dieser zusätzlich noch geöffnet werden muss. Des weiteren ist es mit dieser Vorrichtung schwierig, einen reproduzierbaren Schaum herzustellen, da sich bereits durch eine geringfügige Fehlstellung des Drei-Wege-Hahns der Durchmesser des Durchlasses verändert. Hierdurch kann ein Schaum mit anderer Bläschengröße entstehen. Des weiteren ist die Anordnung der beiden Spritzen in einem 90°-Winkel zueinander nachteilig, da hierdurch das Handling erschwert ist.

Nach der Schaumherstellung ist üblicherweise in beiden Spritzen Schaum vorhanden. Für die Therapie muss bei der in GB 2 369 996 beschriebenen Vorrichtung eine der beiden mit Schaum gefüllten Spritzen von dem Drei-Wege-Hahn abgeschraubt werden. Um den in der zweiten Spritze verbleibenden Schaum erforderlichenfalls zu einem späteren Zeitpunkt zusätzlich zu injizieren, ist es erforderlich, den Drei-Wege-Hahn zuzudrehen, um ein Kontaminieren zu vermeiden. Um sodann den verbleibenden Schaum zu injizieren, muss die erste, soeben für die Therapie genutzte Spritze wieder auf den Drei-Wege-Hahn aufgeschraubt und der Hahn geöffnet werden, um sodann beispielsweise den restlichen Schaum in die Injektionsspritze zu überführen. Es handelt sich hierbei somit um ein äußerst aufwändiges Vorgehen.

Aufgabe der Erfindung ist es, eine Vorrichtung zur Erzeugung eines medizinischen Schaums aus einem gasförmigen und einem flüssigen Medium zu schaffen, mit der hohe Sterilitätsanforderungen erfüllt werden können.

Die Lösung der Aufgabe erfolgt erfindungsgemäß durch die Merkmale der Ansprüche 1 und 12.

Die erfindungsgemäße Vorrichtung zur Erzeugung eines medizinischen Schaums, der insbesondere für die Skleroskopie geeignet ist, weist einen Gasbehälter zur Aufnahme eines sterilen Gases, insbesondere steriler Luft, auf. Ferner ist ein Wirkstoffbehälter zur Aufnahme eines üblicherweise flüssigen Wirkstoffs vorgesehen. Bei den beiden Behältern handelt es sich vorzugsweise um Spritzen, insbesondere Einwegspritzen. Die beiden Behälter sind fluidisch mit einem Verbindungselement verbindbar. Ferner ist eine Fördereinrichtung vorgesehen, um das Gas und den Wirkstoff zwischen den beiden Behältern zur Erzeugung des medizinischen Schaums hin- und herfördern zu können. In einer bevorzugten Ausführungsform umfasst die Fördereinrichtung zwei Förderelemente, wobei jedes Förderelement jeweils mit einem der beiden Behälter verbunden ist. Vorzugsweise handelt es sich bei den Förderelementen um die Spritzenkolben.

Erfindungsgemäß ist das Verbindungselement mit einem der beiden Behälter, vorzugsweise dem Gasbehälter, verbunden. Das Verbinden erfolgt beispielsweise durch verschrauben, insbesondere über ein Luer-Lock. Ebenso kann das Verbindungselement fest mit dem Behälter verbunden, z. B. verklebt oder einstückig ausgebildet sein. Insbesondere kann der die Öffnung der Spritze aufweisende Ansatz als Verbindungselement ausgebildet sein. Hierbei ist das Verbindungselement vorzugsweise von dem Luer-Lock umgeben.

Erfindungsgemäß weist das Verbindungselement zum sterilen Verschließen des Behälters ein insbesondere innenliegendes Verschlusselement auf. Hierdurch ist es möglich, in einem der beiden Behälter, insbesondere in dem Glasbehälter, ein steriles Gas, beispielsweise sterile Luft, vorzusehen, das auf Grund des vorgesehenen Verschlusselementes nicht aus dem Behälter austreten kann. Auch ein ungewolltes Eintreten nicht steriler Luft ist auf Grund des erfindungsgemäßen Vorsehens des Verschlusselementes vorzugsweise vermieden. Das Verbindungselement ist somit in besonders bevorzugter Ausführungsform derart ausgebildet, dass in unverbundenem Zustand, d. h. insbesondere bevor der Gasbehälter zusammen mit dem Verbindungselement mit dem Wirkstoffbehälter verbunden wird, sowohl ein Eintreten als auch ein Austreten von Gas und/ oder Flüssigkeit aus dem mit dem Verschlusselement verschlossenen Behälter vermieden ist. Dies hat den Vorteil, dass eine sehr gute Sterilität des in dem Behälter enthaltenen Mediums sichergestellt ist. Auch ist gewährleistet, dass ein ungewolltes Verändern der Menge in dem Behälter vermieden ist Hierdurch ist eine gute Reproduzierbarkeit des medizinischen Schaums sichergestellt.

Vorzugsweise weist das Verschlusselement einen elastischen Gummistopfen auf. Erfindungsgemäß weist das Verschlusselement einen Schlitz auf, der zum Öffnen des Verschlusselementes dient.
Hierbei ist der Schlitz derart ausgebildet, dass die Schlitzwände in unverbundenem Zustand aneinander anliegen und den Behälter derart dicht verschließen, dass ein Eintreten oder ein Austreten von Gas upd/ oder Flüssigkeit vermieden ist.

Erfindungsgemäß erfolgt das Öffnen des Verschlusselementes automatisch beim Verbinden des Verbindungselementes mit dem zweiten Behälter, insbesondere der zweiten Spritze. Hierdurch ist in bevorzugter Ausführungsform sichergestellt, dass Im Unterschied zu dem Entfernen eines Verschlusselementes In Form eines Deckels oder dgl. bei dem erfindungsgemäßen Öffnen durch das Verbinden eine Kontamination vermieden Ist. Ferner ist kein zusätzlicher Schritt, wie das Entfernen eines Deckels oder das Öffnen eines Hahns, erforderlich. Hierdurch ist es erfindungsgemäß insbesondere möglich, ein Verschlusselement mit deutlich geringerer Kontamlnicrungsgefahr vorzusehen.

Die erfindungsgemäße Vorrichtung zur Erzeugung eines medizinischen Schaums weist insbesondere den Vorteil auf, dass vorzugsweise das sterile Gas bereits steril vorliegt und nicht erst über einen Sterilfilter erzeugt werden muss. Ferner bleibt das Gas auf Grund des automatischen Öffncns steril, so dass ein versehentliches Kontaminieren durch z. B. Aspiration vermieden ist. Des weiteren ist sichergestellt, dass die exakt definierte Gasmenge und somit das Mischungsverhältnis aus Gas und Wirkstoff, beispielsweise durch ungewollten Gasaustritt, nicht verfälscht wird. Hierdurch ist es möglich, einen reproduzierbaren Schaum zu erzeugen und somit einen Standard zu schaffen.

Das Schaffen eines hohen Standards bzw. einer hohen Gleichmäßigkeit des mit Hilfe der erfindungsgemäßen Vorrichtung erzeugbaren Schaums kann weiter dadurch verbessert werden, dass auch der zweite Behälter vorbefüllt ist. Hierzu ist der weitere Behälter, bei dem es sich insbesondere um den Wirkstoffbehälter handelt, mit einem Verschlussteil verschlossen. Das Verschlussteil kann entsprechend dem Verschlusselement, insbesondere als Membran oder Kunststoffstopfen ausgebildet sein. Bei dem Vorsehen von zwei vorbefüllten Behältern, von denen einer mit dem Verbindungselement, das das Verschlusselement aufweist, verschlossen ist, hat ferner den Vorteil, dass ein weiterer Arbeitsschritt, das Befüllen des noch leeren Behälters, üblicherweise des Wirkstoffbehälters, entfällt.

Erfindungsgemäß erfolgt das Öffnen des Verschlusselements durch Durchstoßen einer Membran des Verschlusselementes. Das Durchstoßen der Membran kann durch einen an dem zweiten Behälter, insbesondere an der Spritze vorgesehenen Ansatz, insbesondere den eines Luer-Locks, erfolgen. Erfindungsgemäß erfolgt das Öffnen des Verschlusselementes derart, dass es sich um einen reversiblen Vorgang handelt und das Verschlusselement somit den Behälter in unverbundenem Zustand wieder verschließt. Erfindungsgemäß ist die Membran oder der Kunststoffstopfen hierbei derart ausgebildet, dass er einen Schlitz aufweist, der durch ein rohrförmiges Element auseinander gedrückt werden kann und sich beim Herausziehen des rohrförmigen Elementes wieder verschließt.

Erfindungsgemäß weist das Verbindungselement ein Rohrelement auf. Beim Verbinden des zweiten Behälters mit dem Verbindungselement erfolgt ein Öffnen der Membran durch das Rohr. Hierzu ist das Verschlusselement und/ oder das Rohrelement vorzugsweise in dem Verbindungselement verschiebbar angeordnet. Das Rohrelement ist hierbei vorzugsweise nicht verschiebbar in dem Verbindungselement gehalten, so dass durch Verschieben des Verschlusselementes ein Durchstoßen der Membran durch das Rohrelement erfolgt. Hierbei erfolgt das Verschieben des Verschlusselementes vorzugsweise durch einen Ansatz des Behälters, insbesondere den Luer-Lock-Ansatz einer Spritze.

Sobald die Behälter über das Verbindungselement miteinander verbunden sind, kann das Gas und der Wirkstoff zur Erzeugung des sterilen Schaums zwischen den beiden Behältern, insbesondere den beiden Spritzen, hin- und hergepumpt werden. Hierbei strömt das Gas und der Wirkstoff vorzugsweise durch das Rohrelement. Es erfolgt insbesondere kein Umströmen des Verschlusselementes. Dies hat den Vorteil, dass eindeutig definierte Strömungswege und somit ein eindeutig vorher bestimmbares Strömungsverhalten gegeben sind. Dies erhöht die Reproduzierbarkeit des medizinischen Schaums.

Um ein sicheres Verschließen des ersten Behälters vor dem Verbinden mit dem zweiten Behälter sicherzustellen, ist das Verschlusselement vorzugsweise federbelastet. Beim Öffnen des Verschlusselementes wird das Verschlusselement vorzugsweise gegen die Federkraft gedrückt. Die Federkraft kann beispielsweise durch eine Spiralfeder oder ein anderes elastisches Element hervorgerufen werden. Durch das Vorsehen eines derartigen Verschlusselementes ist gewährleistet, dass die Befüllmenge in dem Behälter konstant bleibt und nicht beispielsweise während des Transportes oder des Handlings verändert wird. Des weiteren ist der Gasbehälter wiederverschließbar und weist eine sehr gute Sterilität auf.

Vorzugsweise weist das Verbindungselement ein Mischelement auf. Besonders bevorzugt ist es hierbei, dass das Rohr, das zum Öffnen der Membran dient, als Mischelement ausgebildet ist. Hierbei ist es ausreichend, ein Röhrchen mit geringem Querschnitt vorzusehen, so dass auf Grund der Querschnittsänderung Turbulenzen entstehen, die zur Durchmischung des Wirkstoffs mit dem Gas dienen. Das Rohrelement, das vorzugsweise aus Edelstahl hergestellt ist, oder eine wirkstoffresistente Beschichtung aufweist, weist beispielsweise einen Innendurchmesser bis 3 mm auf. Eine der Öffnungen des Rohrs kann unmittelbar oder mittelbar durch das Vorsehen eines Zusatzelementes im Querschnitt reduziert sein. Die Querschnittsreduzierung erfolgt vorzugsweise auf einen Querschnitt von 0,3 - 2 mm. Untersuchungen haben gezeigt, dass hierdurch eine sehr gute Qualität an medizinischem Schaum mit sehr guter Reproduzierbarkeit erzeugt werden kann. Zusätzlich können beispielsweise innerhalb des Mischelementes Barrieren, Umlenkelemente und dgl. vorgesehen sein, um die Erzeugung ausreichender Turbulenzen zu gewährleisten.

Die erfindungsgemäße Vorrichtung hat insbesondere den Vorteil, dass es auf Grund der Ausgestaltung des Verbindungselementes beispielsweise nach dem Herstellen des Schaums nicht erforderlich ist, einen Hahn oder dgl. zu schließen, um ein Kontaminieren eines beispielsweise in einem der beiden Spritzen verbleibenden Schaums zu vermeiden. Dies ist nicht erforderlich, da beim Entfernen der Spritze von dem Verbindungselement ein automatisches Verschließen mit Hilfe des Verschlusselementes erfolgt. Zur insbesondere späteren Entnahme des in der Spritze verbliebenen Schaums kann auf einfache Weise ein erneutes sicheres Konnektieren der zur Injektion verwendeten Spritze mit dem Verbindungselement erfolgen. Das Handling der erfindungsgemäßen Vorrichtung ist somit bei Gewährleistung einer hohen Sicherheit sehr einfach.

Ferner betrifft die Erfindung einen Behälter, wie eine Spritze, die insbesondere zur Verwendung in der erfindungsgemäßen Vorrichtung geeignet ist. Der Behälter, der vorzugsweise mit Gas befüllt ist, ist mit einem Verbindungselement, das ein Verschlusselement aufweist, verbunde. Das Verbindungselement, das insbesondere mit einem zweiten Behälter, insbesondere einer zweiten Spritze verbindbar ist, ist vorzugsweise wie vorstehend beschrieben weitergebildet.

Ferner betrifft die Erfindung einen Kit zur Erzeugung eines medizinischen Schaums mit dem vorstehend beschriebenen ersten Behälter, der insbesondere mit Gas befüllt und mit Hilfe des Verbindungselementes verschlossen ist. Ferner weist der Kit einen zweiten Behälter auf , bei dem es sich wie auch bei dem ersten Behälter insbesondere um eine Spritze handelt. Zusätzlich kann der Kit ein Wirkstoffgefäß, wie eine Wirkstoffampulle, aufweisen, in der beispielsweise das Sklerosierungsmittel enthalten ist. Zum Erzeugen des medizinischen Schaums wird der Wirkstoff aus dem Wirkstoffgefäß in den zweiten Behälter gefüllt. Dies erfolgt vorzugsweise durch Einsaugen in den als Spritze ausgebildeten zweiten Behälter. Hierzu ist ggf. zusätzlich eine Nadel in dem Kit vorhanden.

Bei einer alternativen Ausführungsform des Kits ist an Stelle des Wirkstoffgefäßes der zweite Behälter, insbesondere die zweite Spritze, bereits mit Wirkstoff befüllt und mit einem Verschlussteil wie vorstehend beschrieben verschlossen.

Bei einer besonderes bevorzugten Ausführungsform des Kits sind die beiden Behälter, bei denen es sich insbesondere um handelsüblich Spritzen handelt, vorbefüllt und mit Hilfe des Verbindungselemente miteinander verbunden. Hierbei ist die Verbindung jedoch derart, dass das Verschlusselement des Verbindungselementes noch nicht geöffnet ist. Dies kann beispielsweise dadurch erfolgen, dass der zweite Behälter, insbesondere die zweite Spritze, mit Hilfe des Luer-Locks noch nicht vollständig auf das Verbindungselement aufgeschraubt ist. Die Verbindung zwischen den beiden Behältern wird sodann durch vollständiges Eindrehen bzw. Verbinden des zweiten Behälters mit dem Verbindungselement hergestellt.

Ein derartiger Kit weist insbesondere den Vorteil auf, dass der medizinische Schaum sehr schnell hergestellt werden kann. Es sind keine Vorbereitenden Arbeitsschritte, die zeitaufwändig sind, erforderlich. Hierdurch kann die Akzeptanz bei den behandelnden Ärzten erhöht werden. Ferner ist die Gefahr von Kontaminierungen beim Verbinden einzelner Bauteile vermieden.

Nachfolgend wird die Erfindung an Hand einer bevorzugten Ausführungsform unter Bezugnahme auf die anliegenden Zeichnungen näher erläutert.

Es zeigen:
- Fig. 1: eine schematische geschnittene Seitenansicht des Verbindungselementes,
- Fig. 2: eine schematische geschnittene Seitenansicht des Verbindungselementes in mit zwei Behältern verbundenem Zustand,
- Fig. 3: eine schematische geschnittene Teil-Ansicht des Verbindungselementes zusammen mit dem Verschlusselement einer weiteren Ausführungsform,
- Fig. 4: eine schematische Draufsicht der in Fig. 3 dargestellten Ausführungsform und
- Fig. 5 - 7: eine schematische geschnittene Teil-Ansichten des Verbindungselementes zusammen mit dem Verschlusselement weiterer Ausführungsformen.

Ein Verbindungselement 10 weist einen zylindrischen Ansatz 12 mit einem Innengewinde 14 auf. Innerhalb des Ansatzes 12 ist durch ein Rohrelement 16, mit insbesondere kreisförmigem Querschnitt, ein Kanal 18 ausgebildet. In dem Kanal 18 ist ein Rohr 20 angeordnet, dass sich annähernd über die gesamte Länge des Verbindungselementes erstreckt. Das Rohrelement 16 weist an einer Stirnseite 22 eine in den Kanal 18 mündende Öffnung 24 auf.

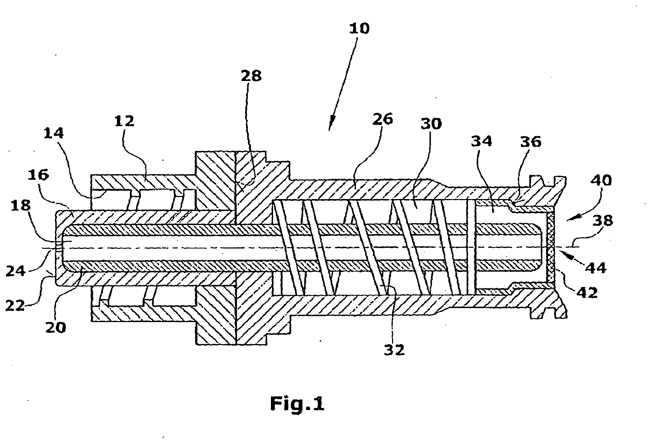

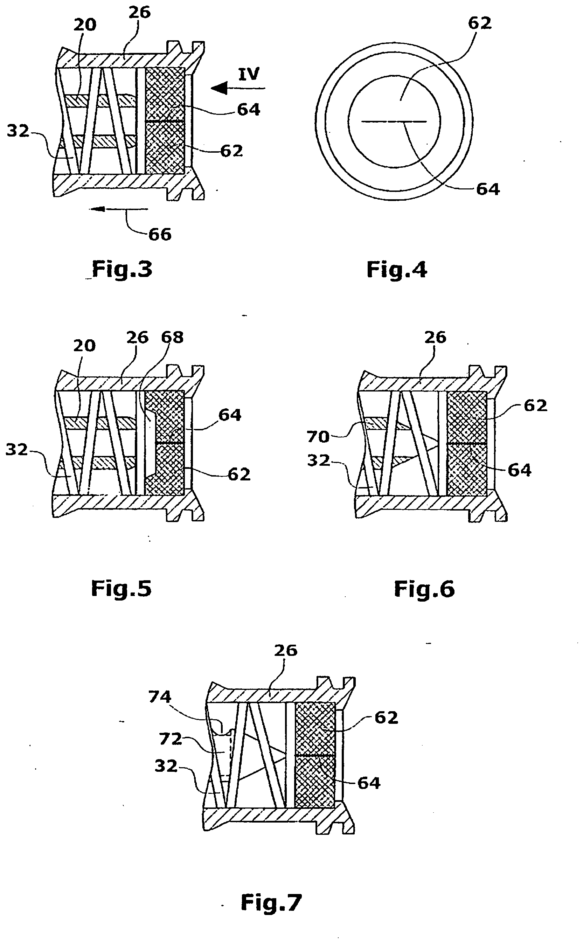

Mit dem Ansatz 12 ist ein Gehäuseelement 26 verbunden. Die Verbindung kann entlang einer Berührungsfläche 28 durch Verkleben erfolgen. Ebenso können die beiden Teile miteinander verschraubt oder auf andere Weise miteinander verbunden sein. In dem Gehäuse 26 ist ein kreiszylinderförmiger Hohlraum 30 ausgebildet. Innerhalb des Hohlraums 30 ist eine Spiralfeder 32 angeordnet, die ein ebenfalls in dem Hohlraum 30 angeordnetes Verschlusselement 34 nach außen gegen einen Anschlag 36, bei dem es sich im dargestellten Ausführungsbeispiel um eine Fase handelt, drückt. Bei dem Verschlusselement 34, das in der dargestellten Ausführungsform eine Membran 42 und eine Hülse aufweist, handelt es sich um ein elastisch verformbares Element, das in zusammengedrückter Form in das Gehäuseelement in Fig. 1 von rechts eingeschoben werden kann, innerhalb des Gehäuses 26 wieder seine ursprüngliche Form annimmt und sodann auf Grund des Anschlags 36 innerhalb des Gehäuses 26 gehalten ist. Ebenso ist es möglich, das Gehäuse 26 zweiteilig auszugestalten, um die Montage des Verschlusselementes 34 zu erleichtern. Hierbei kann das Gehäuse 36 derart getrennt werden, dass das Einführen des Verschlusselementes 34 von der in Fig. 7 linken Seite möglich ist.

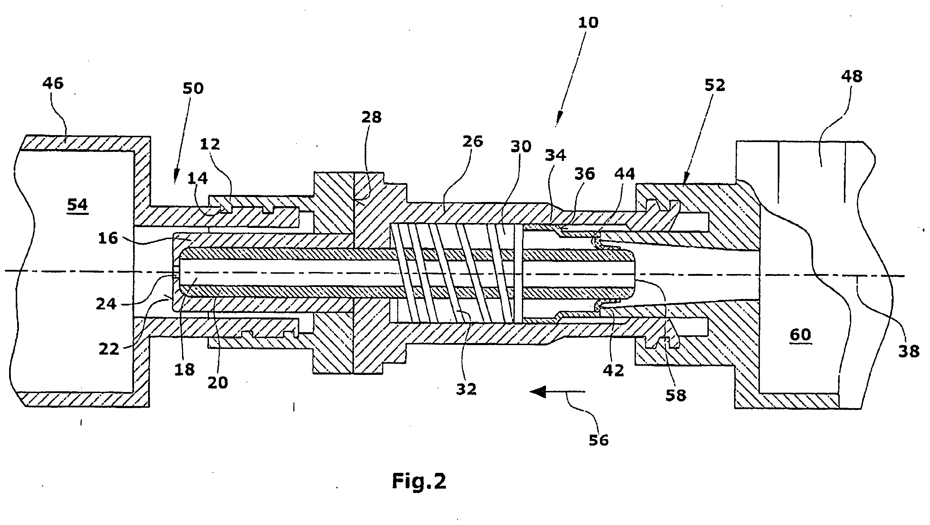

Das Verschlusselement ist zu einer Mittellinie 38 des Verbindungselementes 10, wie auch das Gehäuse 26 und der Ansatz 12, rotationssymmetrisch ausgebildet. Eine Vorderseite 40 des Verschlusselementes 34 ist mit einer Membran 42 verschlossen. Die Membran 42 weist einen Schlitz 44 auf. Der Schlitz 44 ist in der Zeichnung zur Verdeutlichung dargestellt. Tatsächlich liegen die Membranteile in dem in Fig. 1 dargestellten Zustand aneinander an, so dass der Schlitz 44 verschlossen ist, jedoch einfach geöffnet werden kann (Fig. 2).

Das Verbindungselement 10 kann mit einem Gasbehälter 46 und einem Wirkstoffbehälter 48 verbunden werden, wobei es sich bei den beiden Behältern 46, 48 vorzugsweise um herkömmliche Spritzen mit Luer-Lock-Anschlüssen 50 bzw. 52 handelt. Zum Transport sowie vor dem Vermischen des in dem Gasbehälter 46 vorhandenen Gases mit dem in dem Wirkstoffbehälter 48 vorhandenen Wirkstoff ist nur der Gasbehälter 46 mit dem Verbindungselement verbunden. Hierzu wird der Luer-Lock-Anschluss 50 des Gasbehälters 46 in den Ansatz 12 eingeschraubt. Auf Grund der Öffnung 24 besteht eine fluidische Verbindung zwischen dem Innenraum 54 des Gasbehälters 46 und dem Kanal 18, in dem das Rohr 20 angeordnet ist.

Vor dem Einstecken bzw. dem Einschrauben des Flüssigkeitsbehälters 48 bzw. des Luer-Locks 52 ist der Behälter 46 auf Grund des Verschlusselements 34 dicht verschlossen.

Durch Einschrauben bzw. Einstecken des Luer-Locks 52 in das Gehäuse 26 wird das Verschlusselement 34 in Richtung des Pfeils 56 in das Verbindungselement 10 hineingeschoben. Hierbei wird der Schlitz 44 der Membran 42 geöffnet, bzw. Die Membran 42 durchstoßen. Auf Grund der in dem Rohr vorgesehenen Öffnung 58 ist somit ein Innenraum 60 des Wirkstoffbehälters 48 ebenfalls fluidisch mit dem Kanal 18 verbunden.

Durch Betätigen der Spritzenkolben bzw. einer Fördereinrichtung kann der Wirkstoff aus dem Innenraum 60 durch das Rohr 20 bzw. den Kanal 18 in den Innenraum 54 bzw. das Gas aus dem Innenraum 54 durch das Rohr 20 in den Innenraum 60 gepumpt werden. Hierdurch erfolgt ein Durchmischen des Gases mit dem Wirkstoff und sodann ein gemeinsames Hin- und Herpumpen des Gases und des Wirkstoffs zwischen den beiden Räumen 54, 60. Hierdurch entsteht der medizinische Schaum. Das Hin- und Herpumpen des Gases sowie des Wirkstoffs kann durch eine Pumpvorrichtung erfolgen. Dies hat den Vorteil, dass die beispielsweise auf die Spritzenkolben aufgebrachte Kraft sowie die Pumpgeschwindigkeit einstellbar bzw. definiert ist. Hierdurch ist die Standardisierung des erzeugten Schaums weiter verbessert.

Hierbei dient das Rohr 20 als Mischelement und kann ggf. zusätzliche Umlenk- oder Mischelemente im Inneren aufweisen. Umlenk- oder Mischelemente können ferner auch oder zusätzlich am Ein- und/ oder Auslass des Rohrs 20 angeordnet sein. Ggf. können zusätzlich oder anstatt der vorstehend beschriebenen Mischelemente auch Mischelemente in anderen Bereichen der Vorrichtungen, durch die der Wirkstoff und das Gas strömt, vorgesehen sein. Ferner wird die Länge des Rohrs 20 vorzugsweise sinnvoll gewählt, insbesondere empirisch bestimmt. Erfindungsgemäß ist die durch die Öffnung 24 und die Öffnung 58 hervorgerufenen Querschnittsänderung zur Durchmischung ausreichend.

In den Fig. 3 bis 7 sind weitere Ausführungsbeispiel des erfindungsgemäßen Verbindungselementes mit unterschiedlichen Verschließelementen dargestellt. In den Fig. 3 - 7 sind identische oder ähnliche Bauteile mit den selben Bezugszeichen bezeichnet.

Bei der in den Fig. 3 und 4 dargestellten Ausführungsform ist innerhalb des Gehäuses 26 als Verschließelement ein Kunststoff- oder Gummistopfen 62 angeordnet. Der Kunststoffstopfen 62 kann wie vorstehend an Hand des Verschließelementes 34 beschrieben montiert werden. Der Kunststoffstopfen 62 weist einen Schlitz 64 auf, der beim Bewegen des Kunststoffstopfens 62 in Richtung eines Pfeils 66 auseinandergedrückt wird. Wird der Kunststoffstopfen 62 durch die Feder 32 wieder in die in Fig. 3 dargestellte Lage zurückgedrückt, erfolgt ein automatisches erneutes Verschließen des Schlitzes 64.

Bei der in Fig. 5 dargestellten Weiterbildung weist der Kunststoffstopfen 62 zusätzlich eine in Richtung des Rohrs 20 weisende Ausnehmung 68 auf, so dass das Rohr 20 eindeutig geführt ist und ein sicheres Öffnen des Schlitzes gewährleistet ist.

In den in Fig. 6 und 7 dargestellten Ausführungsformen erfolgt das Öffnen des Kunststoffstopfens 62 nicht mit Hilfe eines Rohres 20, sondern mit Hilfe einer Nadel 70 bzw. 72. Hierbei ist die Nadel 70 entweder in Richtung des Stopfens geöffnet, oder die Nadel 72 weist eine seitliche Öffnung 74 auf. Durch das Vorsehen der seitlichen Öffnung 74 werden Turbulenzen erzeugt, die je nach verwendetem Wirkstoff eine verbesserte Schaumerzeugung ermöglichen. Durch das Vorsehen von Nadeln 70, 72 können die Schlitze 64 entfallen.

## Patentansprüche

1. Vorrichtung zur Erzeugung eines Sklerosierungsschaums, mit
einem Gasbehälter (46) zur Aufnahme eines sterilen Gases,
einem Wirkstoffbehälter (48) zur Aufnahme eines Wirkstoffs,
einem Verbindungselement (10) zum Verbinden des Gasbehälters (46) mit dem Wirkstoffbehälter (48) und
einer Fördereinrichtung zum Hin- und Herfördern des Gases und des Wirkstoffs zwischen den beiden Behältern (46, 48) zur Erzeugung des Sklerosierungsschaums,
wobei
das Verbindungselement (10) mit einem der Behälter (46, 48) verbunden ist und zum sterilen Verschließen des Behälters ein Verschlusselement (34, 62) aufweist,
**dadurch gekennzeichnet, dass** die Behälter (46, 48) mit Hilfe des Verbindungselements (10) derart miteinander verbindbar sind, dass das Verschlusselement (34, 62) des Verbindungselements noch nicht geöffnet ist,
wobei das Verschlusselement (34, 62) durch vollständiges Verbinden des Verbindungselementes (10) mit dem zweiten Behälter (48) automatisch öffenbar ist,
wobei das Öffnen durch Durchstoßen einer an dem Verschlusselement (34) vorgesehenen Membran (42) erfolgt,
das Verbindungselement (10) ein Rohr (20) aufweist, das beim vollständigen Verbinden der beiden Behälter (46, 48) die Membran (42) durchstößt,
die Membran des Verschlusselements (34, 62) zum Öffnen einen Schlitz (34) aufweist,
das Öffnen des Verschlusselements (34, 62) derart erfolgt, dass es sich um einen reversiblen Vorgang handelt und das Verschlusselement (34, 62) somit den Behälter in unverbundenem Zustand wieder verschließt und
wobei die Membran (42) derart ausgebildet ist, dass der Schlitz durch das Rohr (20) auseinandergedrückt werden kann und sich beim Herausziehen des Rohrs (20) wieder verschließt.

2. Vorrichtung nach Anspruch 1, **dadurch gekennzeichnet, dass** das Verschlusselement (34, 62) und/ oder das Rohr (20) in dem Verbindungselement (10) verschiebbar angeordnet ist.

3. Vorrichtung nach einem der Ansprüche 1 oder 2, **dadurch gekennzeichnet, dass** das Verschlusselement (34, 62) und/ oder das Rohr (20) federbelastet ist.

4. Vorrichtung nach einem der Ansprüche 1-3, **dadurch gekennzeichnet, dass** das Verbindungselement (10) ein Mischelement (20) aufweist.

5. Vorrichtung nach Anspruch 4, **dadurch gekennzeichnet, dass** das Mischelement (20) das in dem Verbindungselement (10) vorgesehene Rohr (20) ist.

6. Vorrichtung nach einem der Ansprüche 1 - 5, **dadurch gekennzeichnet, dass** die Fördereinrichtung je Behälter (46, 48) ein Förderelement umfasst.

7. Vorrichtung nach einem der Ansprüche 1 - 6, **dadurch gekennzeichnet, dass** das Verschlusselement (34, 62) derart ausgebildet ist, dass in unverbundenem Zustand sowohl ein Eintreten als auch ein Austreten von Gas und/ oder Flüssigkeit aus den Behälter (46, 48) vermieden ist.

8. Vorrichtung nach einem der Ansprüche 1 - 7, **dadurch gekennzeichnet, dass** das Verschlusselement einen elastischen Kunststoffstopfen (62) aufweist.

9. Vorrichtung nach einem der Ansprüche 1 - 8, **dadurch gekennzeichnet, dass** in verbundenem Zustand der Wirkstoff und das Gas durch das Rohrelement (20) strömt.

10. Vorrichtung nach einem der Ansprüche 1 - 9, **dadurch gekennzeichnet, dass** der erste, mit dem Verschlusselement (34, 62) des Verbindungselementes (10) verschlossene Behälter (46) vorbefüllt ist.

11. Vorrichtung nach einem der Ansprüche 1 - 10, **dadurch gekennzeichnet, dass** der zweite Behälter (48) mit einem Verschlussteil verschlossen ist.

12. Vorrichtung zur Erzeugung eines Sklerosierungsschaums, mit
einem Gasbehälter (46) zur Aufnahme eines sterilen Gases,
einem Wirkstoffbehälter (48) zur Aufnahme eines Wirkstoffs,
einem Verbindungselement (10) zum Verbinden des Gasbehälters (46) mit dem Wirkstoffbehälter (48) und
einer Fördereinrichtung zum Hin- und Herfördern des Gases und des Wirkstoffs zwischen den beiden Behältern (46, 48) zur Erzeugung des Sklerosierungsschaums,
wobei
das Verbindungselement (10) mit einem der Behälter (46, 48) verbunden ist und zum sterilen Verschließen des Behälters ein Verschlusselement (34, 62) aufweist,
**dadurch gekennzeichnet, dass** das Verschlusselement (34, 62) durch vollständiges Verbinden des Verbindurigselements (10) mit dem zweiten Behälter (48) automatisch öffenbar ist,
wobei das Öffnen durch Durchstoßen einer an dem Verschlusselement (34) vorgesehenen Membran (42) erfolgt,
das Verbindungselement (10) ein Rohr (20) aufweist, das beim vollständigen Verbinden der beiden Behälter (46, 48) die Membran (42) durchstößt,
die Membran des Verschlusselements (34, 62) zum Öffnen einen Schlitz (34) aufweist,
das Öffnen des Verschlusselements (34, 62) derart erfolgt, dass es sich um einen reversiblen Vorgang handelt und das Verschlusselement (34, 62) somit den Behälter in unverbundenem Zustand wieder verschließt und
wobei die Membran (42) derart ausgebildet ist, dass der Schlitz durch das Rohr (20) auseinandergedrückt werden kann und sich beim Herausziehen des Rohrs (20) wieder verschließt, wobei
der erste Behälter (46) mit Gas befüllt ist und mit dem Verbindungselement (10) verschlossen ist, und der zweite Behälter (48) mit Wirkstoff vorbefüllt und verschlossen ist und der erste Behälter (46) über das Verbindungselement (10) mit dem zweiten Behälter (48) verbunden, das Verschlusselement (34, 62) aber noch ungeöffnet ist.

## Claims

1. A device for producing sclerosing foam, comprising
a gas vessel (46) for holding a sterile gas,
an active agent vessel (48) for holding an active agent,
a connecting element (10) for connecting the gas vessel (46) with the active agent vessel (48), and
a feed means for feeding the gas and the active agent back and forth between the two vessels (46, 48) to produce the sclerosing foam,
wherein
the connecting element (10) is connected with one of the vessels (46, 48) and comprises a closure element (34, 62) for a sterile closure of the vessel,
**characterized in that**
the vessels (46, 48) are adapted to be connected with each other by means of the connecting element (10) such that the closure element (34, 62) of the connecting element is not yet opened, wherein the closure element (34, 62) may be opened automatically by completely connecting the connecting element (10) with the second vessel (48),
wherein opening is effected by penetrating a membrane (42) provided in particular at the closure element (34),
wherein the connecting element (10) comprises a tube (20) which penetrates the membrane (42) when the two vessels (46, 48) are completely connected,
wherein the membrane of the closure element (34, 62) comprises a slit (34) for being opened,
wherein the opening of the closure element (34, 62) is effected such that it is a reversible process and the closure element (34, 62) thus closes the vessel again in the unconnected state, and
wherein the membrane (42) is configured such that the slit can be pushed open by the tube (20) and closes again as the tube (20) is withdrawn.

2. The device of claim 1, wherein the closure element (34, 62) and/or the tube (20) is displaceable within the connecting element (10).

3. The device of one of claims 1 or 2, wherein the closure element (34, 62) and/or the tube (20) is spring-loaded.

4. The device of one of claims 1-3, wherein the connecting element (10) comprises a mixing element (20).

5. The device of claim 4, wherein the mixing element (20) is the tube (20) provided in the connecting element (10).

6. The device of one of claims 1-5, wherein the feed means comprises one feed element per vessel (46, 48).

7. The device of one of claims 1-6, wherein the closure element (34, 62) is configured such that in the unconnected state both an intrusion and an escape of gas and/or liquid into or from the vessels (46, 48) is prevented.

8. The device of one of claims 1-7, wherein the closure element comprises a resilient plastics stopper (62).

9. The device of one of claims 1-8, wherein, in the connected state, the active agent and the gas flow through the tube element (20).

10. The device of one of claims 1-9, wherein the first vessel (46), closed by the closure element (34, 62) of the connecting element (10), is prefilled.

11. The device of one of claims 1-10, wherein the second vessel (48) is closed by a closure member.

12. A device for producing sclerosing foam, comprising
a gas vessel (46) for holding a sterile gas,
an active agent vessel (48) for holding an active agent,
a connecting element (10) for connecting the gas vessel (46) with the active agent vessel (48), and
a feed means for feeding the gas and the active agent back and forth between the two vessels (46, 48) to produce the sclerosing foam,
wherein
the connecting element (10) is connected with one of the vessels (46, 48) and comprises a closure element (34, 62) for a sterile closure of the vessel,
**characterized in that**
the closure element (34, 62) may be opened automatically by completely connecting the connecting element (10) with the second vessel (48), wherein opening is effected by penetrating a membrane (42) provided in particular at the closure element (34),
wherein the connecting element (10) comprises a tube (20) which penetrates the membrane (42) when the two vessels (46, 48) are completely connected,
wherein the membrane of the closure element (34, 62) comprises a slit (34) for being opened,
wherein the opening of the closure element (34, 62) is effected such that it is a reversible process and the closure element (34, 62) thus closes the vessel again in the unconnected state, and
wherein the membrane (42) is configured such that the slit can be pushed open by the tube (20) and closes again as the tube (20) is withdrawn, wherein the first vessel (46) is filled with gas and closed by a connecting element (10), and the second vessel (48) is prefilled with active agent and closed, wherein the first vessel (46) is connected with the second vessel (48) through the connecting element (10), whereas the closure element (34, 62) is still unopened.

## Revendications

1. Dispositif pour produire une mousse sclérosante, comprenant un réservoir de gaz (46) destine à recevoir un gaz stérile,
un réservoir de substance active (48) destiné à recevoir une substance active,
un élément de raccordement (10) destiné à raccorder le réservoir de gaz (46) au réservoir de substance active (48) et
un système de transport pour faire aller et venir le gaz et la substance active entre les deux réservoirs (46, 48), en vue de la production de la mousse sclérosante,
l'élément de raccordement (10) étant relié à l'un des réservoirs (46, 48) et présente un élément obturateur (34, 62) pour assurer une obturation stérile du réservoir,
**caractérisé en ce que**
les réservoirs (46, 48) peuvent être reliés l'un à l'autre au moyen dudit élément de raccordement (10) de sorte que l'élément obturateur (34, 62) dudit élément de raccordement (10) ne soit pas encore ouvert, ledit élément obturateur (34, 62) pouvant être ouvert automatiquement par le raccordement complet dudit élément de raccordement (10) avec le deuxième réservoir (48),
l'ouverture se faisant par transperçage d'une membrane (42) prévue sur l'élément obturateur (34),
l'élément de raccordement (10) présentant un tube (20) qui, lors du raccordement complet des deux réservoirs (46, 48), transperce la membrane (42),
la membrane dudit élément obturateur (34, 62) présente, pour l'ouverture, une fente (34),
l'ouverture dudit élément obturateur (34, 62) est faite de sorte que l'opération soit réversible et ledit élément obturateur (34, 62) referme le réservoir en état non-raccordé, et
la membrane (42) étant conçue de sorte que la fente puisse être poussée par le tube (20) pour s'ouvrir, et se referme après le tube (10) soit retiré.

2. Dispositif selon la revendication 1, **caractérisé en ce que** l'élément obturateur (34, 62) et/ou le tube (20) est monté à coulissement dans l'élément de raccordement (10).

3. Dispositif selon l'une des revendications 1 ou 2, **caractérisé en ce que** l'élément obturateur (34, 62) et/ou le tube (20) est charge par ressort.

4. Dispositif selon l'une des revendications 1 à 3, **caractérisé en ce que** l'élément de raccordement (10) présente un élément mélangeur (20).

5. Dispositif selon la revendication 4, **caractérisé en ce que** l'élément mélangeur (20) est le tube (20) prévu dans l'élément de raccordement (10).

6. Dispositif selon l'une des revendications 1 à 5, **caractérisé en ce que** le système de transport comprend un élément de transport pour chaque réservoir (46, 48).

7. Dispositif selon l'une des revendications 1 à 6, **caractérisé en ce que** l'élément obturateur (34, 62) est conçu de telle façon que, dans l'état non raccordé, une entre aussi bien qu'une sortie de gaz et/ou de liquide des réservoirs (46, 48) soient empêchées.

8. Dispositif selon l'une des revendications 1 à 7, **caractérisé en ce que** l'élément obturateur présente un bouchon élastique (62) en matière plastique.

9. Dispositif selon l'une des revendications 1 à 8, **caractérisé en ce que** dans l'état raccordé, la substance active et le gaz s'écoulent à travers l'élément tubulaire (20).

10. Dispositif selon l'une des revendications 1 à 9, **caractérisé en ce que** le premier réservoir (46), obturé avec l'élément obturateur (34, 62) de l'élément de raccordement (10), est pré-rempli.

11. Dispositif selon l'une des revendications 1 à 10, **caractérisé en ce que** le deuxième réservoir (48) est obturé avec une pièce d'obturation

12. Dispositif pour produire une mousse sclérosante, comprenant un réservoir de gaz (46) destine à recevoir un gaz stérile,
un réservoir de substance active (48) destiné à recevoir une substance active,
un élément de raccordement (10) destiné à raccorder le réservoir de gaz (46) au réservoir de substance active (48) et
un système de transport pour faire aller et venir le gaz et la substance active entre les deux réservoirs (46, 48), en vue de la production de la mousse sclérosante,
l'élément de raccordement (10) étant relié à l'un des réservoirs (46, 48) et présente un élément obturateur (34, 62) pour assurer une obturation stérile du réservoir,
**caractérisé en ce que**
ledit élément obturateur (34, 62) peut être ouvert automatiquement par le raccordement complet dudit élément de raccordement (10) avec le deuxième réservoir (48),
l'ouverture se faisant par transperçage d'une membrane (42) prévue sur l'élément obturateur (34),
l'élément de raccordement (10) présentant un tube (20) qui, lors du raccordement complet des deux réservoirs (46, 48), transperce la membrane (42),
la membrane dudit élément obturateur (34, 62) présente, pour l'ouverture, une fente (34),
l'ouverture dudit élément obturateur (34, 62) est faite de sorte que l'opération soit réversible et ledit élément obturateur (34, 62) referme le réservoir en état non-raccordé, et
la membrane (42) étant conçue de sorte que la fente puisse être poussée par le tube (20) pour s'ouvrir, et se referme après le tube (10) soit retiré, ledit premier réservoir étant rempli de gaz et obturé à l'aide d'un élément de raccordement (10), et le deuxième réservoir (48) étant pré-rempli de substance active et obturé, et le premier réservoir (46) est raccordé au deuxième réservoir (48) par l'intermédiaire de l'élément de raccordement (10), mais l'élément obturateur (34, 62) n'est pas encore ouvert.
